# DEMANDE DE BREVET EUROPEEN

(11) **EP 0 686 394 A1**
(43) Date de publication de la demande: **13.12.1995**
(21) Numéro de dépôt: 95401291.0
(22) Date de dépôt: 02.06.1995
(51) Int. Cl.: A61K 31/335

(54) **Utilisation de dérivés de N-[[5-(napthtalén-1-yl)-1,3-dioxan-2-yl] alkyl] acétamide pour la préparation de médicaments utiles dans le traitement des désordres neurodégénératifs**

(30) Priorité: 08.06.1994 FR 9407021
(71) Demandeur: SYNTHELABO, F-92350 Le Plessis Robinson (FR)
(72) Inventeur: Benavides, Jésus, F-92290 Chatenay Malabry (FR); Duverger, Danielle, F-94550 Chevilly Larue (FR); Evanno, Yannick, F-78830 Bullion (FR); Perrault, Ghislaine, F-75012 Paris (FR); Sevrin, Mireille, F-75014 Paris (FR)
(74) Mandataire: Thouret-Lemaitre, Elisabeth

(57) **Abrégé**

Utilisation des composés de formule (I) 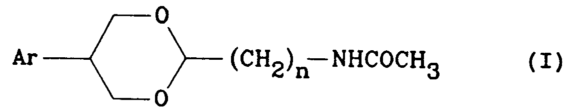
dans laquelle
Ar représente un groupe napthalén-1-yle portant éventuellemnt un substituant choisi parmi les atomes d'halogènes et les groupes méthyle, méthoxy et cyclopropylméthoxy et
n = 1 à 3,
sous forme de stéréoisomères cis, trans ou d'un mélange de ces deux isomères,
pour la préparation de médicaments utiles dans le traitement des désordres neurodégénératifs.

## Description

La présente invention a pour objet l'utilisation de dérivés de *N*-[[5-(naphtalén-1-yl)-1,3-dioxan-2-yl]alkyl]acétamide de formule (I) 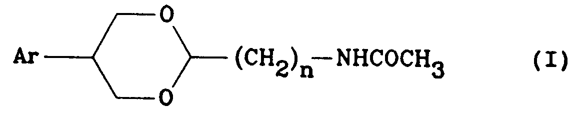
dans laquelle
Ar représente un groupe napthalén-l-yle portant éventuellement un substituant choisi parmi les atomes d'halogènes et les groupes méthyle, méthoxy et cyclopropylméthoxy et
n = 1 à 3,
pour la préparation de médicaments utiles dans le traitement des désordres neurodégénératifs.

Les composés de formule (I) et leur préparation sont décrits dans le brevet européen EP-0461958. Ces composés obtenus par condensation d'un diol avec un acétal, peuvent exister sous la forme de stéréoisomères cis ou trans, la forme trans étant majoritaire lors de la synthèse.
On obtient la forme cis par concentration des eaux mères et recristallisation.

Pour l'utilisation selon l'invention, les composés de formule (I) peuvent avoir la forme de stéréoisomères cis, trans ou d'un mélange de ces deux isomères.

Les composés de formule (I) ont été soumis à des essais pharmacologiques qui ont mis en évidence leurs propriétés neuroprotectrices.

Ainsi les composés utilisables selon l'invention ont été soumis au test de l'ischémie cérébrale globale chez la souris. L'ischémie est due à un arrêt cardiaque induit par une injection intraveineuse rapide de chlorure de magnésium. Dans ce test on mesure le "temps de survie", c'est-à-dire l'intervalle entre le moment de l'injection de chlorure de magnésium et le dernier mouvement respiratoire observable de chaque souris. Ce dernier mouvement est considéré comme l'indice ultime d'une fonction du système nerveux central.
L'arrêt respiratoire apparaît approximativement 21 secondes après l'injection de chlorure de magnésium.
Des souris mâles (SWISS OF₁ IFFA CREDO) sont étudiées par groupes de 10. Elles sont nourries et abreuvées ad libitum avant les essais. Le temps de survie est mesuré 10 minutes après l'administration intrapéritonéale ou 5 minutes après l'administration intraveineuse dans la veine caudale, des composés de formule (I). Les résultats sont donnés sous la forme de la différence entre le temps de survie mesuré dans un groupe de 10 souris ayant reçu le composé et le temps de survie mesuré dans un groupe de 10 souris ayant reçu le liquide véhicule.
Les rapports entre le temps de survie et la dose du composé sont enregistrés graphiquement selon une courbe semilogarithmique.
Cette courbe permet le calcul de la "dose efficace 3 secondes" (DE_{3sec}), c'est-à-dire la dose (en mg/kg) qui produit une augmentation de 3 secondes du temps de survie par rapport au groupe témoin de 10 souris traitées par le véhicule.
Une augmentation de 3 secondes du temps de survie est à la fois significative statistiquement et reproductible.
A titre d'exemple, après injection par voie intrapéritonéale, la DE_{3sec} du *trans-N*-[3-[5-(6-méthoxynaphtalén-1-yl)-1,3-dioxan-2-yl]propyl]acétamide dans ce test est de 11,4 mg/kg, la DE_{3sec} du *cis-N*-[3-[5-(6-méthoxynaphtalén-1-yl)-1,3-dioxan-2-yl]propyl]acétamide dans ce test est de 6,1 mg/kg et la DE_{3sec} du *trans-N*-[3-[5-[6-(cyclopropylméthoxy)naphtalén-1-yl]-1,3-dioxan-2-yl]propyl]acétamide dans ce test est de 6,1 mg/kg.
Par voie intraveineuse, après solubilisation dans une solution de 2-hydroxypropyl-β-cyclodextrine, la DE_{3sec} du *trans-N*-[3-[5-(6-méthoxynaphtalén-1-yl)-1,3-dioxan-2-yl]propyl]acétamide dans ce test est de 2,25 mg/kg.

L'activité neuroprotectrice des composés de formule (I) a été également démontrée dans un modèle d'ischémie focale permanente par occlusion intraluminale de l'artère cérébrale moyenne chez le rat, selon une méthode analogue à celle décrite dans *Stroke*, **20**, 84-91 (1989).
Sous anesthésie au méthohexital sodique, on ligature l'artère ptérygopalatine, l'artère carotidienne commune et l'artère carotidienne externe gauche; on introduit un fil de nylon dans l'artère carotidienne interne sur une longueur d'environ 18 mm correspondant à la distance qui sépare la naissance de l'artère carotidienne interne de celle de l'artère cérébrale moyenne.
Les composés sont administrés 10 minutes, 2 heures 30 et 5 heures après l'occlusion, aux doses de 0,3 ; 1 ; 3 et 10 mg/kg par voie intrapéritonéale. 24 heures après l'occlusion de l'artère cérébrale moyenne, on sacrifie les animaux et on prélève le cerveau. On évalue le volume de l'infarctus cérébral à partir de la mesure de la surface de la nécrose sur 6 coupes coronales colorées par le chlorure de 2,3,5-triphényltétrazolium.
A titre d'exemple le *trans-N*-[3-[5-(6-méthoxynaphtalén-1-yl)-1,3-dioxan-2-yl]propyl]acétamide réduit le volume de l'infarctus cérébral d'environ 30% à la dose de 1 mg/kg.

Les résultats de ces essais montrent que les composés de formule (I) ont des propriétés neuroprotectrices et peuvent être utilisés pour la préparation de médicaments utiles dans le traitement et la prévention des désordres cérébrovasculaires d'origine ischémique/hypoxique (infarctus cérébral, traumatismes crâniens et médullaires, arrêt cardiaque ou respiratoire, attaques ischémiques transitoires, asphyxie périnatale), du glaucome, des maladies neurodégénératives progressives (démences séniles de type Alzheimer, maladies de Parkinson et de Huntington, atrophie olivo-ponto-cérébelleuse, sclérose latérale amyotrophique, maladies neurodégénératives d'origine virale, etc...), et dans la prévention des accidents ischémiques cérébraux associés à la chirurgie cardiaque et vasculaire et à la thérapie endovasculaire.

A cet effet ils peuvent être présentés sous toutes formes pharmaceutiques adaptées à l'administration entérale ou parentérale, en association avec des excipients appropriés.
Ils peuvent être présentés par exemple sous forme de comprimés, dragées, gélules, capsules, suppositoires, solutions ou suspensions buvables ou injectables.
Certaines de ces formes pharmaceutiques peuvent contenir un complexe constitué par un composé de formule (I) avec une cyclodextrine ou un de ces dérivés.
Ces formes pharmaceutiques sont dosées pour permettre une administration journalière de 1 à 1000 mg de substance active.

## Revendications

1. Utilisation des composés de formule (I) 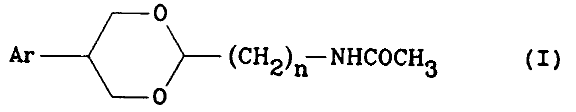 dans laquelle
Ar représente un groupe napthalén-1-yle portant éventuellemnt un substituant choisi parmi les atomes d'halogènes et les groupes méthyle, méthoxy et cyclopropylméthoxy et n = 1 à 3,
sous forme de stéréoisomères cis, trans ou d'un mélange de ces deux isomères,
pour la préparation de médicaments utiles dans le traitement des désordres neurodégénératifs.

2. Utilisation selon la revendication 1 caractérisée en ce que le composé de formule (I) est le *trans*-*N*-[3-[5-(6-méthoxynaphtalén-1-yl)-1,3-dioxan-2-yl]propyl]acétamide.

3. Utilisation selon la revendication 1 caractérisée en ce que le composé de formule (I) est le *cis*-*N*-[3-[5-(6-méthoxynaphtalén-1-yl)-1,3-dioxan-2-yl]propyl]acétamide.

4. Utilisation selon la revendication 1 caractérisée en ce que le composé de formule (I) est le *trans*-*N*-[3-[5-[6-(cyclopropylméthoxy)naphtalén-1-yl)-1,3-dioxan-2-yl]propyl] acétamide.

5. Utilisation selon l'une quelconque des revendications 1 à 4 caractérisée en ce qu'on utilise un complexe constitué par le principe actif et une cyclodextrine ou un de ses dérivés.
